# FASCICULE DE BREVET EUROPEEN

(11) **EP 2 459 122 B1**
(45) Date de publication et mention de la délivrance du brevet: **10.09.2014**
(21) Numéro de dépôt: 10752858.0
(22) Date de dépôt: 30.07.2010
(51) Int. Cl.: A61F 2/08

(54) **LIGAMENT PROTHETIQUE POUR FIXATION TRANSVERSALE ET PROCEDE DE CONFECTION**
PROTHETISCHES BAND FÜR TRANSVERSALE FIXATION UND HERSTELLUNGSVERFAHREN
PROSTHETIC LIGAMENT FOR TRANSVERSE FIXATION, AND PRODUCTION METHOD

(30) Priorité: 31.07.2009 FR 0903790
(43) Date de publication de la demande: 06.06.2012
(73) Titulaire: L.A.R.S. - laboratoire d'application et de Recherche Scientifique, 21560 Arc-sur-Tille (FR)
(72) Inventeur: BRULEZ, Bernard, F-52400 Bourbonne Les Bains (FR); LABOUREAU, Jacques-Philippe, F-06530 Le Tignet (FR)
(74) Mandataire: Oudin, Stéphane
(86) Numéro de dépôt international: PCT/FR2010/000557
(87) Numéro de publication internationale: WO 2011/012783

(56) Documents cités:
- EP-A- 0 145 492
- EP-A- 1 493 404
- WO-A-89/01320
- GB-A- 2 276 823
- US-A- 5 263 984

## Description

L'invention concerne un ligament prothétique destiné à être mis en place selon une technique de fixation transversale ainsi qu'un procédé de confection d'un tel ligament ; ce ligament est utilisable dans la plastie du genou pour le remplacement des ligaments croisés antérieurs ou postérieurs.

On connaissait déjà un certain nombre de ligaments artificiels destinés au remplacement de ligaments articulaires. Parmi ceux-ci il existe des ligaments obtenus par pliage ou roulage convoluté de bandes de textile d'un matériau biocompatible tissé ou tricoté de manière à laisser libre, ou au contraire à lier entre eux, les fils techniques longitudinaux du ligament. Ces ligaments connus présentent généralement une forme globale de cylindre allongé comprenant entre deux parties externes intra-osseuses une partie médiane intra-articulaire. Des exemples de tels ligaments sont également donnés dans les brevets français FR 2.755.846 et FR 2.688.690.

La technique chirurgicale habituelle de fixation de ces ligaments artificiels ou des reconstructions ligamentaires biologiques consistait à percer un tunnel osseux dans le fémur et dans le tibia puis à bloquer dans ces tunnels la construction ligamentaire à ses extrémités par une vis à interférence fixée frontalement sur l'os. Pour permettre leur mise en place dans les tunnels osseux, les extrémités des ligaments sont munies de fils de traction. En outre, on a pu parfois observer un glissement de la vis de fixation sur le ligament prothétique au niveau de l'os du fémur avec cette technique.

Les chirurgiens ont donc développé une nouvelle technique d'implantation des ligaments avec un système de fixation transversale, dans lequel le tunnel fémoral est un trou borgne sur lequel débouche un tunnel latéral permettant l'insertion d'une vis de blocage de l'extrémité fémorale du ligament, permettant ainsi une fixation en suspension et éventuellement en compression du ligament. Cette nouvelle technique de fixation des ligaments pour la plastie ligamentaire offre, outre une meilleure résistance du ligament à la traction, l'avantage d'un traumatisme moindre pour le patient puisqu'il n'y a plus de percement de tunnel débouchant dans l'os du fémur et, améliore en outre la colonisation fibroblastique des prothèses biologiques ainsi fixées.

Les brevets EP 1 493 404 et EP 0 145 492 décrivent des ligaments artificiels munis d'oeillets à l'extrémité de leurs parties externes intra-osseuses ; ces oeillets servent à recevoir un dispositif de fixation du type vis ou bouton à la surface externe de l'os, le reste du ligament passant dans des tunnels osseux traversants. Ces ligaments ne sont donc ni conçus ni adaptés à la technique de fixation transversale.

La présente invention a donc pour objet de fournir un ligament prothétique artificiel adapté à ces nouvelles techniques de fixation transversales ainsi qu'un procédé de confection d'un tel ligament.

A cet égard, l'invention a pour objet un ligament prothétique pour le remplacement d'un ligament naturel articulaire comportant une première partie extrême intra-osseuse, dite tibiale, et une seconde partie extrême intra-osseuse, dite fémorale, les deux parties extrêmes intra-osseuses encadrant une partie médiane intra-articulaire, ledit ligament étant obtenu par pliage ou roulage convoluté de bandes de textile d'un matériau biocompatible comportant des fils techniques longitudinaux remarquable en ce que la première partie extrême intra-osseuse se présente sous la forme de deux brins cylindriques et en ce que la seconde partie extrême intra-osseuse forme une boucle reliée à la première partie extrême intra-osseuse par la partie médiane intra-articulaire qui est constituée d'au moins deux faisceaux de fils techniques, chacun des faisceaux étant lié à l'une de ses extrémités à la première partie extrême intra-osseuse et à l'autre de ses extrémités à la seconde partie extrême intra-osseuse.

On comprend bien l'avantage de cette construction en boucle avec une partie médiane intra-articulaire à double faisceaux qui permet d'utiliser une laize présentant globalement un nombre de fils techniques plus faible, comparativement aux ligaments prothétiques de l'art antérieur, au niveau de la première partie extrême intra-osseuse qui correspond à la partie fixée dans le tibia, tandis que le dédoublement des fils dans la partie médiane intra-articulaire améliore d'autant plus la résistance à la traction et que la boucle ainsi formée peut être facilement insérée et contenue dans le demi-tunnel fémoral pratiqué dans cette technique de fixation latérale.

De préférence et avantageusement, les fils techniques sont liés entre eux dans au moins l'une des première et seconde parties extrêmes intra-osseuses et sont libres dans la partie intra-articulaire.

Selon le mode d'exécution de l'invention, la boucle du ligament est ouverte.

L'invention a également pour objet un procédé de confection d'un ligament adapté à la technique de fixation transversale avec tunnel latéral.

Ce procédé est remarquable en ce qu'il comporte au moins les étapes de fournir une laize présentant cinq secteurs successifs : un premier secteur extrême tramé dont la longueur correspond à celle du tunnel tibial, un premier secteur médian intra-articulaire, un second secteur extrême tramé qui va servir à former la seconde partie extrême intra-osseuse du ligament fini, un second secteur médian intra-articulaire et un dernier secteur extrême tramé dont la longueur correspond à celle du tunnel tibial ; puis, de rouler la laize sur elle-même en cylindre, en formant des extrémités coniques, après avoir coupé les extrémités en biseau ; ensuite, de fixer le roulage convoluté par au moins une couture longitudinale réalisée sur au moins les premier et dernier secteurs extrêmes tramés; enfin, de plier le roulage en cylindre ainsi obtenu en deux selon sa longueur, symétriquement, de sorte à former la boucle ouverte.

D'autres avantages et caractéristiques ressortiront mieux de la description qui va suivre d'un exemple de réalisation donné à titre non limitatif d'un ligament selon l'invention et du procédé de confection associé à un tel ligament, en référence aux dessins annexés sur lesquels :
- la figure 1 est une représentation schématique et en perspective vue du dessus du ligament selon l'invention,
- les figures 2A à 2D représentent les étapes schématiques du procédé de confection du ligament représenté à la figure 1.

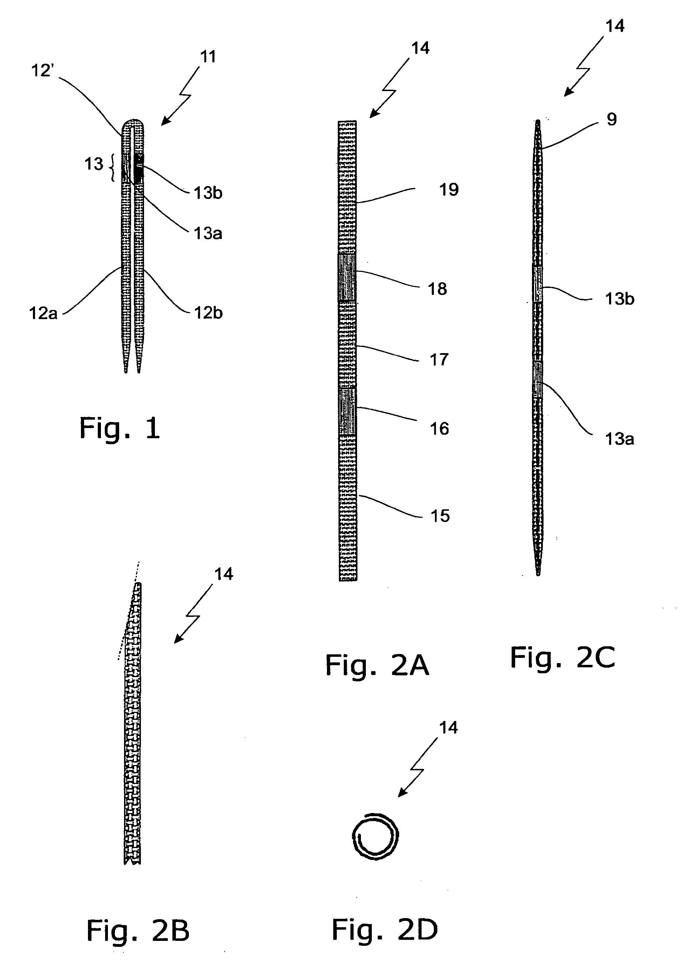

En référence aux figures 1 et 2A à 2D, on décrira un mode d'exécution du ligament 11 selon l'invention, dit à boucle ouverte.

Ce mode d'exécution présente de nombreux avantages : tout d'abord, sa fabrication est simple. De surcroit, le diamètre final des faisceaux étant étroit, sa pose nécessite le percement d'un tunnel de faible diamètre, ce qui est nettement préférable au plan chirurgical.

Selon une caractéristique essentielle, en référence à la figure 1, la boucle formée par la seconde partie extrême intra-osseuse 12', dite extrémité fémorale, est ouverte. Ainsi, la première partie extrême intra-osseuse 12, dite extrémité tibiale, est constituée de deux brins cylindriques 12a, 12b.

Les deux parties extrêmes intra-osseuses 12, 12' sont reliées l'une à l'autre via une partie médiane intra-articulaire 13 constituée de deux faisceaux 13a, 13b de fils techniques. Les fils techniques de la partie médiane intra-articulaire 13 sont libres tandis qu'ils sont liés entre eux dans les parties extrêmes intra-osseuses 12, 12'. Il va bien entendu de soi que l'on pourra réaliser des ligaments 11 selon l'invention présentant des fils techniques liés entre eux dans la partie médiane intra-articulaire 13 sans sortir du cadre de la présente invention.

Ce ligament 11 comporte donc cinq tronçons consécutifs : un premier tronçon tramé, correspondant au brin cylindrique 12a, un second tronçon de fils libres, correspondant au faisceau 13a, un troisième tronçon tramé, correspondant à la première partie extrême intra-osseuse 12, un quatrième tronçon de fils libres, correspondant au second faisceau 13b et un cinquième tronçon tramé, correspondant au brin cylindrique 12b.

De préférence, le ligament 11 présente un axe de symétrie, de sorte que les deux brins cylindriques 12a, 12b sont de même longueur et respectivement, les deux faisceaux 13a, 13b.

Ce ligament 11 est spécifiquement conçu pour être mis en place par poussée dans les tunnels osseux au moyen d'un guide de pose objet d'une demande de brevet non encore publiée déposée par la demanderesse. Il ne comporte donc pas de fils de traction.

On décrira maintenant un exemple de procédé de confection de ce ligament 11 à boucle ouverte selon l'invention en référence à la séquence illustrée aux figures 2A à 2D.

Le ligament 11 est fabriqué à partir d'une laize 14 unique représentée à la figure 2A.

Cette laize 14 présente cinq secteurs successifs : un premier secteur extrême 15 tramé dont la longueur correspond à celle du tunnel tibial, un premier secteur médian intra-articulaire 16, un second secteur extrême 17 tramé qui va servir à former la seconde partie extrême intra-osseuse 12' du ligament 11 fini, un second secteur médian intra-articulaire 18 et un dernier secteur extrême 19 tramé dont la longueur correspond à celle du tunnel tibial.

A titre d'exemple non limitatif, la longueur du second secteur extrême tramé 17 sera de 35 mm environ, celle des premier et second secteurs médians intra-articulaires 16, 18 de 28 mm environ et celle des premier et dernier secteurs extrêmes tramés 15, 19 respectivement de 175 mm environ.

Selon la variante de réalisation préférentielle représentée sur les figures 1 et 2A à 2D, les premier et second secteurs médians intra-articulaires 16, 18 sont chacun composés uniquement d'un écheveau de fils techniques longitudinaux libres, tandis que dans les secteurs extrêmes tramés 15, 17, 19, les fils techniques sont liées entre eux en une trame tissée ou tricotée.

La première étape du procédé, non représentée sur les figures, consiste donc à découper et/ou à tricoter la laize 14 aux dimensions requises.

Ensuite, on roule la laize 14 sur elle-même, en une rotation d'un tour et trois quarts de tour environ, par exemple dans le sens inverse des aiguilles d'une montre comme représenté à la figure 2D. Lors du roulage en cylindre, on prendra soin de rouler les extrémités libres des premier et derniers secteurs extrêmes tramés 15, 19 en forme de cône, après avoir coupé les extrémités en biseau comme illustré à la figure 2B ; puis, en référence à la figure 2C, on fixe le roulage convoluté par une couture longitudinale 9 réalisée sur tous les secteurs extrêmes tramés 15, 17, 19, et au moins sur le premier et le dernier secteurs extrêmes 15, 19.

Enfin, on plie le roulage en cylindre ainsi obtenu en deux selon sa longueur, symétriquement, de sorte à former la boucle ouverte.

Il va bien entendu de soi que le procédé que l'on vient de donner à titre d'exemple n'est qu'une méthode préférentielle d'obtention du ligament pour fixation transversale selon l'invention et que l'Homme de l'Art pourra y apporter des aménagements, notamment dans l'ordre des étapes ou le nombre de coutures, sans pour autant sortir du cadre de la présente invention.

En outre, si la technique de pose spécifique de ce nouveau ligament prothétique permet d'améliorer sensiblement sa colonisation tissulaire, on pourra bien entendu appliquer des traitements spécifiques aux matériaux constitutifs dudit ligament afin d'améliorer sa biocompatibilité. En particulier et avantageusement, on utilisera le procédé de traitement de surface décrit dans le brevet européen EP 1.599.238 au nom du demandeur.

Enfin, il va de soi que, si le nouveau ligament artificiel et son procédé de fabrication sont particulièrement destinés au remplacement du ligament croisé antérieur du genou, cette structure de ligament peut être utilisée pour le remplacement d'autres ligaments et les exemples que l'on vient de donner ne sont donc que des illustrations particulières en aucun cas limitatives des domaines d'application de l'invention.

## Revendications

1. Ligament (11) prothétique pour le remplacement d'un ligament naturel articulaire comportant une première partie extrême intra-osseuse (12), dite tibiale, et une seconde partie extrême intra-osseuse (12'), dite fémorale, les deux parties extrêmes intra-osseuses (12, 12') encadrant une partie médiane intra-articulaire (13), ledit ligament (11) étant obtenu par pliage ou roulage convoluté de bandes de textiles d'un matériau biocompatible comportant des fils techniques longitudinaux, **caractérisé en ce que** la première partie extrême intra-osseuse (12) se présente sous la forme de deux brins(12a, 12b) cylindriques et **en ce que** la seconde partie extrême intra-osseuse (12') forme une boucle ouverte reliée à la première partie extrême intra-osseuse (12) par la partie médiane intra-articulaire (13) qui est constituée d'au moins deux faisceaux (13a, 13b) de fils techniques, chacun des faisceaux (13a, 13b) étant lié à l'une de ses extrémités à la première partie extrême intra-osseuse (12) et à l'autre de ses extrémités à la seconde partie extrême intra-osseuse (12'), le ligament (11) étant ainsi constitué de cinq tronçons consécutifs : un premier tronçon tramé, correspondant à un premier brin cylindrique (12a), un second tronçon de fils libres, correspondant à un premier faisceau (13a), un troisième tronçon tramé, correspondant à la deuxième partie extrême intra-osseuse (12'), un quatrième tronçon de fils libres, correspondant au second faisceau (13b) et un cinquième tronçon tramé, correspondant au second brin cylindrique (12b).

2. Ligament (11) selon la revendication 1 **caractérisé en ce que** la seconde partie extrême intra-osseuse (12') comporte un nombre de fils techniques longitudinaux égal à la somme de celui des faisceaux (13a, 13b) de fils de la partie médiane intra-articulaire (13).

3. Ligament (1, 11) selon l'une quelconque des revendications précédentes **caractérisé en ce que** les fils techniques sont liés entre eux dans au moins l'une des première et seconde parties extrêmes intra-osseuses (12, 12') et sont libres dans la partie médiane intra-articulaire (13).

4. Ligament (11) selon l'une quelconque des revendications 1 à 3 **caractérisé en ce que** le ligament (11) présente un axe de symétrie, de sorte que les deux brins cylindriques (12a, 12b) sont de même longueur et respectivement, les deux faisceaux (13a, 13b).

5. Procédé de confection d'un ligament (11) selon la revendication 1 **caractérisé en ce qu'**il comporte au moins les étapes de fournir une laize (14) présentant cinq secteurs successifs : un premier secteur extrême (15) tramé dont la longueur correspond à celle du tunnel tibial, un premier secteur médian intra-articulaire (16), un second secteur extrême (17) tramé qui va servir à former la seconde partie extrême intra-osseuse (12') du ligament (11) fini, un second secteur médian intra-articulaire (13) et un dernier secteur extrême (19) tramé dont la longueur correspond à celle du tunnel tibial ; puis, de rouler la laize (14) sur elle-même en cylindre en formant des extrémités coniques, après avoir coupé les extrémités en biseau ; ensuite, de fixer le roulage convoluté par au moins une couture longitudinale (9) réalisée sur au moins les premier et dernier secteurs extrêmes tramés (15, 17); enfin, de plier le roulage en cylindre ainsi obtenu en deux selon sa longueur, symétriquement, de sorte à former la boucle ouverte.

## Patentansprüche

1. Prothetisches Band (11) für den Austausch eines natürlichen Gelenkbandes, umfassend einen ersten intraossären Endteil (12), genannt tibial, und einen zweiten intraossären Endteil (12), genannt femoral, wobei die zwei intraossären Endteile (12, 12') einen mittleren intraartikulären Teil (13) einfassen, wobei das Band (11) durch eingerolltes Biegen oder Walzen von Textilbändern aus einem biokompatiblem Material erhalten wird, umfassend längliche technische Garne, **dadurch gekennzeichnet, dass** der ersten intraossäre Endteil (12) die Form von zwei zylindrischen Strängen (12a, 12b) aufweist, und dadurch, dass der zweite intraossäre Endteil (12') eine offene Schlinge bildet, die mit dem ersten intraossären Endteil (12) durch den mittleren intraartikulären Teil (13) verbunden ist, der aus mindestens zwei Bündeln (13a, 13b) aus technischen Garnen besteht, wobei jedes der Bündel (13a, 13b) an einem seiner Enden mit dem ersten intraossären Endteil (12) und am anderen seiner Enden mit dem zweiten intraossären Endteil (12') verbunden ist, wobei das Band (11) somit aus fünf aufeinander folgenden Abschnitten besteht, einem ersten eingeschossenen Abschnitt, der einem ersten zylindrischen Strang (12a) entspricht, einem zweiten Abschnitt aus freien Garnen, die einem ersten Bündel (13a) entsprechen, einem dritten eingeschossenen Abschnitt, der dem zweiten intraossären Endteil (12') entspricht, einem vierten Abschnitt aus freien Garnen, der dem zweiten Bündel (13b) entspricht, und einem fünften eingeschossenen Abschnitt, der dem zweiten zylindrischen Strang (12b) entspricht.

2. Band (11) nach Anspruch 1, **dadurch gekennzeichnet, dass** der zweite intraossäre Endteil (12') eine Anzahl von länglichen technischen Garnen umfasst, die gleich der Summe desjenigen der Bündel (13a, 13b) von Garnen des mittleren intraartikulären Teils (13) ist.

3. Band (1, 11) nach einem der vorhergehenden Ansprüche, **dadurch gekennzeichnet, dass** die technischen Garne untereinander in mindestens einem des ersten und des zweiten intraossären Endteils (12, 12') verbunden sind und im mittleren intraartikulären Teil (13) frei sind.

4. Band (11) nach einem der Ansprüche 1 bis 3, **dadurch gekennzeichnet, dass** das Band (11) eine Symmetrieachse aufweist, so dass die zwei zylindrischen Stränge (12a, 12b) die gleiche Länge aufweisen wie jeweils die zwei Bündel (13a, 13b).

5. Verfahren zur Herstellung eines Bandes (11) nach Anspruch 1, **dadurch gekennzeichnet, dass** es mindestens die Schritte des Bereitstellens einer Bahnbreite (14) umfasst, die fünf aufeinander folgende Abschnitte aufweist: einen ersten eingeschossenen Endabschnitt (15), dessen Länge derjenigen des Tibialtunnels entspricht, einen ersten mittleren intraartikulären Abschnitt (16), einen zweiten eingeschossenen Endabschnitt (17), der dazu dient, den zweiten intraossären Endteil (12') des endgültigen Bandes (11) zu bilden, einen zweiten mittleren intraartikulären Abschnitt (18) und einen letzten eingeschossenen Endabschnitt (19), dessen Länge derjenigen des Tibialtunnels entspricht; dann des Rollens der Bahnbreite (14) auf sich selbst zu einem Zylinder durch Bilden von konischen Enden, nachdem die Enden mit abgeschrägter Kante geschnitten wurden; dann des Befestigens der eingerollten Walze durch mindestens ein längliches Nähen (9), das auf mindestens dem ersten und dem letzen eingeschossenen Endabschnitt (15, 17) durchgeführt wird; schließlich des Biegens der so erhaltenen Walze zu einem Zylinder symmetrisch der Länge nach in zwei Teile, um die offene Schlinge zu bilden.

## Claims

1. Prosthetic ligament (11) for replacing a natural joint ligament comprising a first, or tibial, intraosseous end portion (12), and a second, or femoral, intraosseous end portion (12'), both intraosseous end portions (12, 12') encompassing an intra-articular median portion (13), said ligament (11) being obtained by folding or convoluted rolling of textile strips of a biocompatible material comprising longitudinal technical yarns, **characterised in that** the first intraosseous end portion (12) is in the form of two cylindrical strands (12a, 12b) and **in that** the second intraosseous end portion (12') forms an open loop connected to the first intraosseous end portion (12) by the intra-articular median portion (13) consisting of at least two bundles (13a, 13b) of technical yarns, each of the bundles (13a, 13b) being connected at one of the ends thereof to the first intraosseous end portion (12) and at the other end thereof to the second intraosseous end portion (12'), the ligament (11) thus consisting of five consecutive sections: a first woven section, corresponding to a first cylindrical strand (12a), a second free yarn section, corresponding to a first bundle (13a), a third woven section, corresponding to the second intraosseous end portion (12'), a fourth free yarn section, corresponding to the second bundle (13b) and a fifth woven section, corresponding to the second cylindrical strand (12b).

2. Ligament (11) according to claim 1, **characterised in that** the second intraosseous end portion (12') comprises a number of longitudinal technical yarns equal to the sum of that of the bundles (13a, 13b) of yarns of the intra-articular median portion (13).

3. Ligament (1, 11) according to any of the above claims, **characterised in that** the technical yarns are interconnected in at least one of the first and second intraosseous end portions (12, 12') and are free in the intra-articular median portion (13).

4. Ligament (11) according to any of the claims 1 to 3, **characterised in that** the ligament (11) has an axis of symmetry, such that the two cylindrical strands (12a, 12b) are of the same length and respectively the two bundles (13a, 13b).

5. Method for producing a ligament (11) according to claim 1, **characterised in that** it comprises at least the steps for providing a strip (14) having five successive sectors: a first woven end sector (15), the length whereof corresponds to that of the tibial tunnel, a first intra-articular median sector (16), a second woven end sector (17) used to form the second intraosseous end portion (12') of the finished ligament (11), a second intra-articular median sector (18) and a final woven end sector (19), the length whereof corresponds to that of the tibial tunnel; then, rolling the strip (14) onto itself in a cylinder forming conical ends, after cutting a bevelled edge on the ends; then, securing the convoluted roll with at least one longitudinal stitch (9) produced on at least the first and final woven end sectors (15, 17); finally, folding the cylinder roll obtained in two along the length thereof, symmetrically, so as to form the open loop.
